# EUROPÄISCHE PATENTANMELDUNG

(11) **EP 1 867 651 A1**
(43) Veröffentlichungstag der Anmeldung: **19.12.2007**
(21) Anmeldenummer: 07114437.2
(22) Anmeldetag: 29.10.2004
(51) Int. Cl.: C07F 5/02, C07D 409/14

(54) **Halbleitende Schichten enthaltend lineare organische Thiophen-Phenylen-Oligomere**

(30) Priorität: 12.11.2003 DE 10353094
(62) Teilanmeldung aus: 04025731.3
(71) Anmelder: H.C. Starck GmbH, 38642 Goslar (DE)
(72) Erfinder: Kirchmeyer, Stefan, Dr., 51375 Leverkusen (DE); Ponomarenko, Sergei, Dr., 107589 Moskau (RU)
(74) Vertreter: Clauswitz, Kai-Uwe Wolfram

(57) **Zusammenfassung**

Die Erfindung betrifft neue halbleitende Schichten enthaltend lineare organische Thiophen-Phenylen-Oitgomere der allgemeinen Formel (I) 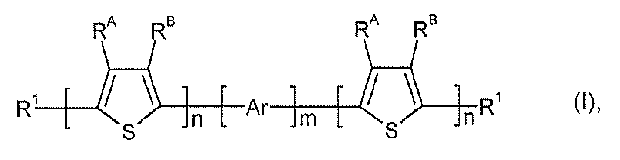 wobei die Verbindungen gemäß Formel (I) unter Verwendung von Thiophen-Pinakoilnboronsäureestern hergestellt werden.

## Beschreibung

Die Erfindung betrifft halbleitende Schichten enthaltend lineare organische Thiophen-Phenylen-Oligomere, deren Verwendung sowie ein Verfahren zu deren Herstellung.

Das Feld molekularer Elektronik hat sich in den letzten 15 Jahren mit der Entdeckung organischer leitender und halbleitender Verbindungen rapide entwickelt. In dieser Zeit wurden eine Vielzahl von Verbindungen gefunden, die halbleitende oder elektrooptische Eigenschaften aufweisen. Wichtige Vertreter halbleitender Verbindungen sind neben Oligothiophenen auch beispielsweise Thiophen-Phenylen-Cooligomere. Solche Verbindungen sind derzeit für Anwendungsgebiete wie organische Feld-Effekt-Transistoren (OFET's), organische Lumineszenzdioden (OLED's), Sensoren und photovoltaische Elemente von großem Interesse.

Die Herstellung von Thiophen-Phenylen-Cooligomere, im Folgenden auch als Thiophen-Phenylen-Oligomere bezeichnet, kann mittels unterschiedlicher, dem Fachmann bekannten Kupplungsreaktionen erfolgen. Dass die Eignung der Endprodukte für den Einsatz in der Halbleitertechnik wesentlich von deren Reinheit abhängt und die nachteiligen Auswirkungen verunreinigter Halbleiter auf die Eigenschaften, wie beispielsweise ein schlechtes "On/Off Ratio" in OFETs, der daraus hergestellten elektronischen Anwendungen sind allgemein bekannt (Handbook of Oligo- and Polythiophenes, ed. by D. Fischou, Wiley-VCH, Weinheim, S. 469-471).

Hotta et al. beschreibt eine Reihe von Phenylen-Oligothiophenen und deren Herstellung über Grignard-Reaktionen oder Suzuki-Kupplung, wobei jedoch so stark verunreinigte Produkte gebildet werden, dass nach Aufreinigung hohe Ausbeuteverluste zu verzeichnen sind (J. Heterocyclic Chem. 2000, 37, 281-286; J. Heterocyclic Chem. 2000, 37, 25-29, Synt. Met. 1999, 101, 551-552). Die Suzuki-Kupplung erfolgt in allen Fällen zwischen Aryl-Boronsäuren und Thienylhalogeniden. Die Eignung von Oligothiophenen mit Phenyleinheiten als Endgruppen als lichtemittierende Schichtmaterialien in OLEDs wurde von Hotta et al. in Synt. Met. 1999, 106, 39-40 beschrieben.

Katz et al. beschrieb kürzlich weitere Thiophen-Phenylen-Oligomere und deren Eignung als oligomere Halbleiter für OFET's. Unglücklicherweise konnte auch hier das am besten geeignete 1,4-Bis(5-hexyl-2,2'-bithien-5-yl)phenyl durch Kupplung von zinnorganischen Thiophen-Verbindungen mit Dihalogenarylverbindungen lediglich in einer extrem niedrigen Ausbeute von 10 % hergestellt werden, so dass diese Methode zur Herstellung größerer Mengen an Oligomeren nicht geeignet ist (chem. Mater. 2001, 13, 4686-4691). Katz et al. beschreibt zudem die Synthese von 1,4-Bis(2,2'-bithien-5-yl)phenyl und 1,4-Bis(4-hexyl-2,2'-bithien-5-yl)phenyl über Suzuki-Kupplung entsprechender Thiophen-Phenylen-Thiophen-Dihalogenide mit Monothiophen-Boronsäuren. Nachteilig bei dieser Methode ist jedoch, dass eine zentrale Thiophen-Phenylen-Thiophen-Dihalogenideinheit durch aufwändige Kupplungsreaktionen hergestellt werden muss. Zudem wird Thiophenboronsäuren eine geringe Stabilität zugeschrieben (Gronowitz et.al., Heterocycles 1990, 30, 645-658; Lehn et al., Eur. Chem. J. 1996, 2, 1399-14.06). Beides resultiert in hohen Ausbeuteverlusten und/oder einer größeren Menge von Verunreinigungen.

In US-A 2002/0114973 werden weitere Thiophen-Phenylen-Oligomere beschrieben, wobei jedoch weiterhin der Nachteil besteht, dass mit zunehmender Kettenlänge zunehmend viele Kupplungsschritte zum Aufbau des Moleküls erforderlich sind, was weiterhin das Problem des hohen Ausbeuteverlustes und größerer Mengen an Verunreinigungen, die über aufwändige und verlustreiche Aufreinigung beseitigt werden müssen, bestehen lässt. Auch in US-A 2002/0114973 wird die Herstellung der Thiophen-Phenylen-Oligomere mittels Grignard-Reaktionen und/oder Suzuki-Kupplung unter Einsatz von Thiophenboronsäure beschrieben.

Es bestand daher weiterhin Bedarf an einem Verfahren zur Herstellung halbleitender organischer Thiophen-Phenylen-Oligomere, bei denen die Endprodukte über möglichst wenige Zwischenprodukte und in hoher Reinheit und guter Ausbeute erhalten werden. Vorteilhafterweise sollte sich ein solches Verfahren zur Herstellung solcher Oligomeren unabhängig von deren Kettenlänge anwenden lassen.

Die Aufgabe der vorliegenden Erfindung bestand somit darin, ein solches Verfahren bereitzustellen.

Überraschend wurde nun gefunden, dass eine Suzuki-Kupplung in der als bororganische Verbindung ein Thiophenpinakolinboronsäureester mit mehr als einer Thiopheneinheit eingesetzt wird, diese Anforderungen erfüllt.

Gegenstand der vorliegenden Erfindung ist daher ein Verfahren zur Herstellung von Verbindungen der allgemeinen Formel (I), 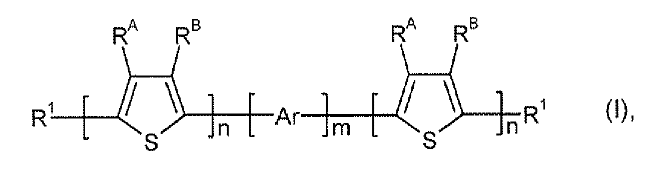 insbesondere nach dem Prinzip der Suzuki-Kupplung, worin
- m: für eine ganze Zahl von 1 bis 5, bevorzugt für 1, 2 oder 3, besonders bevorzugt für 1 steht,
- Ar: für gegebenenfalls substituiertes 1,4-Phenylen und/oder 2,7-Fluorenylen steht,
- R¹: für H oder eine gegebenenfalls substituierte lineare oder verzweigte C₁-C₂₀-Alkylgruppe, bevorzugt eine C₁-C₁₂-Alkylgruppe, besonders bevorzugt eine Ethyl-, n-Butyl, n-Hexyl-, n-Octyl, n-Decyl oder n-Dodecylgruppe, oder eine gegebenenfalls durch ein(e) oder mehrere O- oder S-Atome, Silylen-, Phosphonoyl- oder Phosphorylgruppen unterbrochene lineare C₁-C₂₀-Alkylgruppe, bevorzugt C₁-C₁₂-Alkylgruppe, oder eine C₃-C₂₀-Alkenylgruppe, bevorzugt eine C₁-C₁₂-Alkenylgruppe, besonders bevorzugt eine Allyl-, Butenyl-, Pentenyl-, Hexenyl-, Heptenyl-, Octenyl-, Decenyl-, Undecenyl- oder Dodecenyl-Gruppesteht,
- R^{A}, R^{B}: unabhängig voneinander jeweils für H oder eine gegebenenfalls substituierte und/oder gegebenenfalls mit bis zu 10 Sauerstoffatomen unterbrochene lineare oder verzweigte C₁-C₂₀-Alkylgruppe, bevorzugt für eine C₁-C₁₂-Alkylgruppe, besonders bevorzugt für eine Methyl-, Ethyl-, Propyl-, Butyl-, Pentyl- oder Hexylgruppe, eine gegebenenfalls substituierte C₁-C₂₀-Alkoxygruppe, bevorzugt für eine C₁-C₆-Alkoxygruppe, besonders bevorzugt für eine Methoxygruppe, oder gemeinsam für eine gegebenenfalls substituierte C₁-C₆-Dioxyalkylengruppe, bevorzugt für eine 3,4-Alkylendioxygruppe mit bis zu 6 Kohlenstoffatomen in der Alkylengruppe, beispielsweise eine 3,4-Ethylendioxygruppe oder 3,4-Propylendioxygruppe, stehen und
- n: für 2, 3 oder 4 steht,
dadurch gekennzeichnet, dass eine Verbindung der allgemeinen Formel (II), 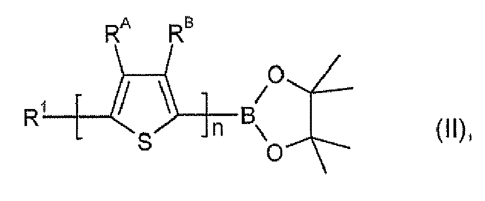 worin
n, R¹, R^{A} und R^{B} die oben für die allgemeine Formel (I) genannte Bedeutung haben.
mit einer Verbindung der allgemeinen Formel (III), 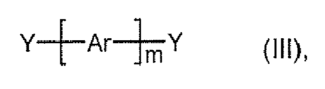 worin
- m und Ar: die für die allgemeine Formel (I) genannte Bedeutung haben und worin
- Y: für einen Rest Cl, Br, I oder O-SO₂-R^{C} steht, wobei R^{C} für einen Phenyl-, Tolyl-, Methyl- oder Trifluormethylrest steht,
gegebenenfalls in Anwesenheit von Katalysatoren, gegebenenfalls in Lösung und gegebenenfalls bei erhöhter Temperatur umgesetzt wird.

Dabei können die n Thiopheneinheiten in den Verbindungen der allgemeinen Formeln(I) oder (II) gleich oder verschieden sein, d.h. die Substituenten R^{A} können in den einzelnen Thiopheneinheiten gleich oder verschieden sein und die Substituenten R^{B} können in den einzelnen Thiopheneinheiten gleich oder verschieden sein.

In einer bevorzugten Ausführungsform der Erfindung stehen R^{A} und R^{B} für H.

Für den Fall, dass R¹ für eine C₃-C₂₀-Alkenylgruppe steht, ist die Doppelbindung der C₃-C₂₀-Alkenylgruppe bevorzugt endständig (vgl. z.B. Formel II-a in Beispiel 1).

In der allgemeinen Formeln (I) und (II) und in weiteren Formeln dieser Anmeldung ist die Strukturformel des Pinakolinboronsäurerestes 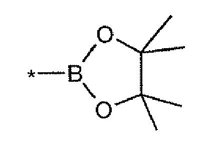 als vereinfachte Form der Strukturformel 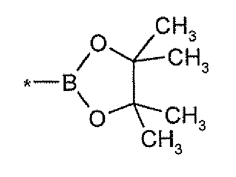 zu verstehen.

Als Substituenten für den Rest Ar kommen beispielsweise lineare oder verzweigte C₁-C₂₀-Alkylreste, bevorzugt C₁-C₁₂-Alkylreste, oder durch ein(e) oder mehrere O-Atome unterbrochene lineare C₁-C₂₀-Alkylreste in Frage. An den 2,7-Fluorenyleneinheiten sitzen (einer oder mehrere) gegebenenfalls vorhandene Substituenten bevorzugt an der 9-Position. Besonderes bevorzugte Substituenten 1,4-Phenylen-Ringe sind Methyl-, Ethyl-, Propyl-, Butyl-, Pentyl- oder Hexyl-Gruppen. Weitere besonderes bevorzugte Substituenten für Phenyl-Gruppen sind Methoxy-, Ethoxy-, Propoxy-, Buthoxy-, Pentoxy-, Hexyloxy- oder Heptyloxy-Gruppen. Ganz besonderes bevorzugter Substituent ist Wasserstoff.

Bevorzugt ist das neue Verfahren zur Herstellung von Verbindungen der allgemeinen Formel (I-a) 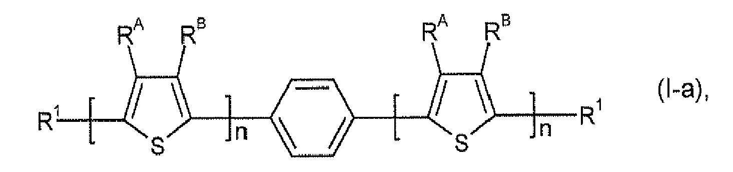 worin
n, R¹, R^{A} und R^{B} die oben für die allgemeine Formel (I) genannte Bedeutung haben,
wobei eine Verbindung der allgemeinen Formel (II) mit einer Verbindung der allgemeinen Formel (III-a) 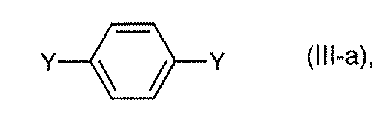 worin
- Y: die oben für die allgemeine Formel (III) genannte Bedeutung hat,
umgesetzt wird.

Beispielhaft für bevorzugt hergestellte Verbindungen der allgemeinen Formel (I-a) sind 5-Alkyl-5'-[4-(5'-alkyl-2,2'-bithien-5-yl)phenyl]-2,2"-bithiophen und 5-Alkyl-5"-[4-(5"-alkyl-2,2':5',2"-terthien-5-yl)phenyl]-2,2'-.5',2"-terthiophen, insbesondere 5-Decyl-5"-[4-(5"-decyl-2,2': 5',2"-terthien-5-yl)phenyl]-2,2':5',2"-terthiophen, 5-Hexyl-5"-[4-(5"-hexyl-2,2':5',2"-terthien-5-yl)phenyl]-2,2':5',2"-terthiophen, 5-Ethyl-5"-[4-(5"-Ethyl-2,2':5',2"-terthien-5-yl)phenyl]-2,2':5',2"-terthiophen, 5-Decyl-5'-[4-(5'-Decyl-2,2'-bithien-5-yl)phanyl]-2,2"-bithiophen, 5-Hexyl-5'-[4-(5'-hexyl-2,2'-bithien-5-yl)phenyl]-2,2"-bithiophen und 5-Etyl-5'-[4-(5-ethyl-2,2'-bithien-5-yl)phenyl]-2,2"-bithiophen, genannt.

Solche Verbindungen sind bereits generell in US 6,355,365 B1 und US 2002/0114973 A1 beschrieben. Jedoch können sie nach dem neuen Verfahren erstmals in besonderer Reinheit, d.h. beispielsweise ohne Verunreinigungen durch strukturelle Isomere, z.B. durch 3,5- bzw. 2,4-verknüpfte Thiopheneinheiten, oder homologe Verbindungen, und nach einfacher einstufiger Umsetzung von Verbindungen der allgemeinen Formel (II) mit Aryldihalogeniden erhalten werden. In US 2002/0114973 sind zur Herstellung aufwändige mehrstufige Verfahren beschrieben, die in verminderter Ausbeute und/oder Reinheit der Zielverbindungen resultieren.

Bevorzugt wird das erfindungsgemäße Verfahren in einer Variante der Suzuki-Kupplung durchgeführt. Die Suzuki-Kupplung, d.h. die Umsetzung von Arylhalogeniden und bororganischen Verbindungen ist z.B. in Suzuki et al., Chem. Rev. 1995, 95, 2457-2483 beschrieben. In einer bevorzugten Ausführungsform wird das neue Verfahren gemäß einer Variante dieser Suzuki-Kupplung durchgeführt, wobei Aryl- bzw. Heteroarylhalogenide und Verbindungen der allgemeinen Formel (I) gegebenenfalls in Anwesenheit wenigstens einer Base und/oder wenigstens eines Katalysators, der ein Metall der VIII. Nebengruppe des Periodensystems der Elemente, im Folgenden kurz als Metall der VIII. Nebengruppe bezeichnet, enthält, umgesetzt werden.

Die besonders bevorzugte Ausführungsform des Verfahrens (Suzuki-Kupplung) wird bei einer Temperatur von +20°C bis +200°C, bevorzugt von +40°C bis +150°C, besonderes bevorzugt von +80°C bis +130°C, in einem organischen Lösungsmittel oder Lösungsmittelgemisch durchgeführt.

Als Katalysatoren, kommen prinzipiell alle Verbindungen in Frage, die ein Metall der VIII. Nebengruppe des Periodensystems der Elemente, bevorzugt Pd, Ni oder Pt, besonders bevorzugt Pd, enthalten. Der oder die Katalysator(en) werden bevorzugt in Mengen von 0,05 Gew.-% bis 10 Gew.-%, besonders bevorzugt von 0,5 Gew.-% bis 5 Gew.-%, bezogen auf das Gesamtgewicht der zu kuppelnden Verbindungen verwendet. Besonders geeignete Katalysatoren sind Komplexverbindungen von Metallen der VIII. Nebengruppe, insbesondere Komplexe des Palladium(0), die an Luft stabil sind, Pd-Komplexe die sich leicht mit Organometallreagenzien (z.B. Lithiumalkylverbindungen oder magnesiumorganische Verbindungen) oder Phosphinen zu Palladium(0)-Komplexen reduzieren lassen, oder Palladium(2)-Komplexen gegebenenfalls unter Zusatz von PPh₃ oder anderen Phosphinen. Beispielsweise können PdCl₂(PPh₃)₂, PdBr₂(PPh₃)₂ oder Pd(OAc)₂ oder Mischungen aus diesen Verbindungen unter Zusatz von PPh₃ eingesetzt werden. Bevorzugt wird Pd(PPh₃)₄ ohne oder unter Zusatz von Phosphinen, in einer bevorzugten Ausführungsform ohne Zusatz von Phosphinen, eingesetzt, welches in preiswerter Form zur Verfügung steht. Als Phosphine werden bevorzugt PPh₃, PEtPh₂, PMePh₂, PEt₂Ph oder PEt₃, besonders bevorzugt PPh₃, eingesetzt.

Es ist jedoch auch möglich als Katalysatoren Palladiumverbindungen ohne Phosphinzusatz einzusetzen, beispielsweise Pd(OAc)₂.

Als Base können beispielsweise Hydroxide, z.B. NaOH, KOH, LiOH, Ba(OH)₂, Ca(OH)₂, Alkoxide, z.B. NaOEt, KOEt, LiOEt, NaOMe, KOMe, LiOMe, Alkalimetallsalze von Carbonsäuren, z.B. Natrium-, Kalium- oder Lithiumcarbonat, -hydrogencarbonat, -acetat, -citrat, -acetylacetonat, -glycinat, oder andere Carbonate, z.B. Cs₂CO₃ oder Tl₂CO₃, Phosphate, z.B. Natriumphosphat, Kaliumphosphat oder Lithiumphosphat, oder Mischungen aus diesen eingesetzt werden. Bevorzugt wird Natriumcarbonat eingesetzt. Die Basen können als Lösungen in Wasser oder als Suspensionen in organischen Lösungsmitteln, wie Toluol, Dioxan oder DMF, eingesetzt werden. Bevorzugt sind Lösungen in Wasser, da die erhaltenen Produkte aufgrund ihrer geringen Löslichkeit in Wasser so vom Reaktionsgemisch leicht abgetrennt werden können.

Es ist auch möglich weitere Salze, wie beispielsweise LiCl oder LiBr, als Hilfsmittel einzusetzen.

Als organische Lösungsmittel kommen grundsätzlich alle Lösungsmittel oder Lösungsmittelgemische in Frage, welche mit den Pinakolinboronsäureestern der allgemeinen Formel (II) nicht reagieren. Dies sind in der Regel Verbindungen, welche keine Halogenatome oder keine gegenüber Thiophen-Pinakolinboronsäureestern reaktive Wasserstoffatome aufweisen. Geeignete Lösungsmittel sind beispielsweise Alkane wie Pentan, Hexan und Heptan, Aromaten wie Benzol, Toluol und Xylole, Ethergruppen enthaltende Verbindungen wie Dioxan, Dimethoxyethan und Tetrahydrofuran und polare Lösungsmittel wie Dimethylformamid oder Dimethylsulfoxid. Bevorzugt werden im neuen Verfahren Aromaten als Lösungsmittel eingesetzt. Ganz besonders bevorzugt ist Toluol. Es ist auch möglich, als Lösungsmittel Mischungen aus zwei oder mehreren dieser Lösungsmittel einzusetzen.

Die Aufarbeitung der Reaktionsmischung erfolgt nach an sich bekannten Verfahren, z.B. durch Verdünnen, Fällen, Filtration, Extraktion, Waschen, Rekristallisation aus geeigneten Lösungsmitteln, Chromatographie und/oder Sublimation. Beispielsweise kann eine Aufarbeitung in der Weise erfolgen, dass die Reaktionsmischung nach vollständiger Reaktion in ein Gemisch aus saurem (Eis-)Wasser, z.B. hergestellt aus 1 molarer Salzsäure, und Toluol gegossen, die organische Phase abgetrennt, mit Wasser gewaschen, das als Feststoff enthaltene Produkt abfiltriert, mit Toluol gewaschen und anschließend im Vakuum getrocknet wird. Die Verbindungen der allgemeinen Formel (I) können bereits ohne weitere anschließende Reinigungsprozesse in hoher Qualität und Reinheit erhalten werden und sind halbleitend. Es ist jedoch möglich, diese Produkte nach bekannten Verfahren, z.B. durch Rekristallisation, Chromatographie oder Sublimation weiter zu reinigen.

Die in diesem Verfahren eingesetzten Aryldihalogenverbindungen der allgemeinen Formel (III) können nach an sich bekannten Verfahren hergestellt werden. Die Herstellung der Pinakolinboronsäureester der allgemeinen Formel (II) wird beispielsweise in Feast et al, J. Mater. Chem. 2003, 13, 1269-1273 beschrieben, wobei allerdings Zinnorganische Verbindungen als Ausgangsverbindungen eingesetzt werden. Aufgrund ihrer gesundheitlichen Bedenklichkeit wäre es jedoch vorteilhaft, den Einsatz solcher zinnorganischen Verbindungen zu vermeiden.

Alternativ können die Verbindungen der allgemeinen Formel (II) nach verschiedenen Verfahren hergestellt werden, die dem Fachmann grundsätzlich bekannt sind. Beispielsweise ist es möglich, die Verbindungen der allgemeinen Formel (II) durch Umsetzung von Arylhalogeniden und Bis-(pinakolato)diboran durch metallkatalysierte Kupplung herzustellen, wie sie beispielsweise in WO-A 01/29051 A1 und Tetrahedron Lett. 2002, S. 5649 beschrieben ist. Weiterhin ist es möglich die Verbindungen der allgemeinen Formel (II) durch Kupplung von Oligothiophenhalogeniden mit Pinakolboran herzustellen. (J. Org. Chem. 1997, B.62, S. 6458; J. Organomet. Chem. 2001, B. 640, S. 197; Chem. Commun. 2002, S. 1566). Diese Reaktionen führen jedoch zu Produkten, die Diaryle als Nebenprodukte enthalten, welche von den Pinakolinboronsäureestern der allgemeinen Formel (II) und besonders von den erfindungsgemäß herzustellenden Thiophen-Phenylen-Oligomeren der allgemeinen Formel (I) schlecht zu trennen sind.

Bevorzugt werden die Verbindungen der allgemeinen Formel (II) daher durch Umsetzung von metallorganischen Verbindungen, z.B. magnesiumorganischen Verbindungen (z.B. Grignard-Verbindungen) oder lithiumorganischen Verbindungen, mit Pinakolinboronsäureestern hergestellt. Dies ist insofern bevorzugt, da bei dieser Reaktion überraschend keine Diaryle als Nebenprodukte gebildet werden.

Weiterhin Gegenstand der vorliegenden Erfindung ist daher ein Verfahren zur Herstellung der Verbindungen der allgemeinen Formel (II) 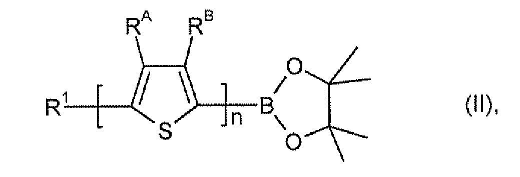 dadurch gekennzeichnet, dass Verbindungen der allgemeinen Formel (IV) 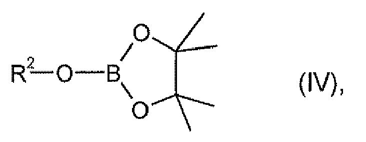 worin
- R²: für einen linearen oder verzweigten C₁-C₂₀-Alkylrest steht,
mit Verbindungen der allgemeinen Formel (V) 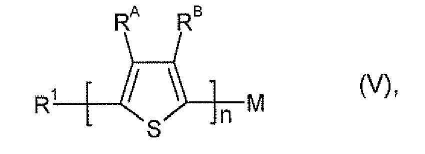 worin
n, R¹, R^{A} und R^{B} die oben für die allgemeine Formel (I) genannte Bedeutung haben und
- M: für LiX oder MgX steht, wobei X für Cl, Br, I, bevorzugt für Br steht,
umgesetzt werden.

Bevorzugte Alkoxypinakolinboronsäureester der allgemeinen Formel (IV) sind dabei solche, worin R² für einen linearen oder verzweigten C₁-C₁₂-Alkylrest steht, besonders bevorzugt für einen Rest aus der Reihe Methyl, Ethyl, n-Propyl, i-Propyl, n-Butyl, t-Butyl, i-Butyl, n-Pentyl oder n-Hexyl. In bevorzugten Ausführungsformen des erfindungsgemäßen Verfahrens werden 2-Isopropoxy-4,4,5,5-tetramethyl-1,3,2-dioxaborolan oder Methoxy-4,4,5,5-tetramethyl-1,3,2-dioxaborolan eingesetzt. Diese beiden Verbindungen haben den Vorteil, dass sie kommerziell zu erwerben sind.

Die Ausgangsverbindungen, wie Grignard- oder lithiumorganische Verbindungen der allgemeinen Formel (V) können nach bekannten Methoden hergestellt werden (vgl. beispielsweise J. Mat. Chem. 2003, 13, 197) oder sind käuflich verfügbar.

Das erfindungsgemäße Verfahren wird so durchgeführt, dass die Umsetzung der metallorgansichen Verbindungen der allgemeinen Formel (V) mit den Alkoxypinakolinboronsäureestern der allgemeinen Formel (IV) bei einer Temperatur von -100°C bis +50°C, bevorzugt von -80°C bis +20°C in einem organischen Lösungsmittel oder Lösungsmittelgemisch erfolgt. Als organische Lösungsmittel oder Lösungsmittelgemische kommen im Prinzip sämtliche Lösungsmittel oder Lösungsmittelgemische in Frage, welche mit den Verbindungen der allgemeinen Formel (IV) und (V) nicht reagieren. Dies sind in der Regel Lösungsmittel, welche keine Halogenatome oder keine gegenüber lithiumorganischen Verbindungen oder Grignard-Verbindungen reaktiven Wasserstoffatome bzw. Carbonylgruppen aufweisen. Geeignete Lösungsmittel sind beispielsweise Alkane wie z.B. Pentan, Hexan und Heptan, Aromaten wie z.B. Benzol, Toluol und Xylole, sowie Ethergruppen enthaltende Verbindungen wie z.B. Diethylether, tert.-Butylmethylether, Dioxan und Tetrahydrofuran. Bevorzugt werden in dem Verfahren Lösungsmittel eingesetzt, die Ethergruppen enthalten. Ganz besonders bevorzugt ist Tetrahydrofuran. Es ist allerdings auch möglich, als Lösungsmittel Mischungen aus zwei oder mehreren der vorgenannten Lösungsmittel einzusetzen. Beispielsweise können Mischungen aus dem bevorzugt verwendeten Lösungsmittel Tetrahydrofuran und Alkanen, z.B. Hexan verwendet werden. Solche Mischungen können beispielsweise in kommerziell erhältlichen Lösungen von Ausgangsprodukten wie lithiumorganischen Verbindungen enthalten sein.

Die Verbindungen der allgemeinen Formel (II) lassen sich nach bekannten Methoden der Aufarbeitung z.B. durch einfachen Abfiltrieren aus der Reaktionslösung isolieren und gegebenenfalls weiter reinigen. Als Reinigungsmethoden sind beispielsweise geeignet Rekristallisation, Sublimation oder Chromatographie. Die Verbindungen der allgemeinen Formel (II) können entweder in isolierter Form oder direkt in der Reaktionslösung ("in situ") hergestellt und ohne weitere Aufarbeitung eingesetzt werden.

Beispielhaft für Verbindungen der allgemeinen Formel (II) seien 4,4,5,5-Tetramethyl-2-[5'-(10-undecenyl)-2,2'-bithien-5-yl]-1,3,2-dioxaborolan (II-a), 2-(5"-Decyl-2,2':5',2"-terthien-5-yl)-4,4,5,5-tetramethyl-1,3,2-dioxaborolan (II-b), 4,4,5,5-Tetramethyl-2-[5'-hexyl-2,2'-bithien-5-yl]-1,3,2-dioxaborolan (II-c), 2-(5"-Hexyl-2,2':5',2"-terthien-5-yl)-4,4,5,5-tetramethyl-1,3,2-dioxaborolan (IId) genannt:

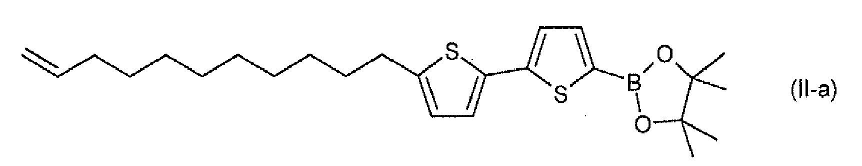

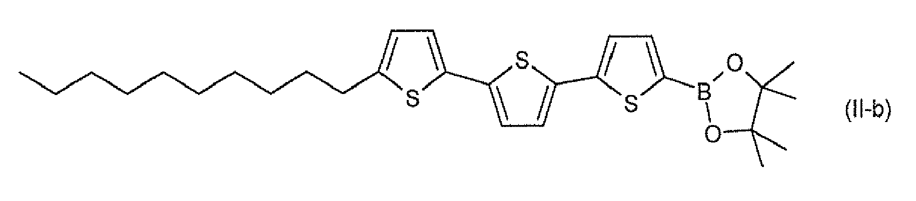

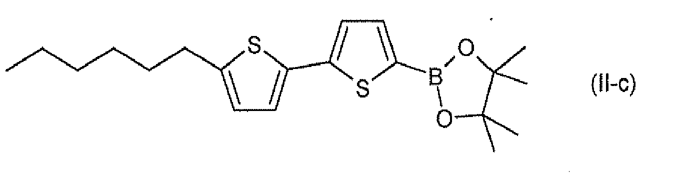

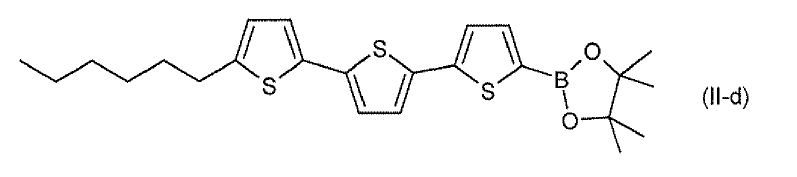

Bevorzugt werden die Verbindungen der allgemeinen Formel (II) in einer Reinheit von mindestens 90 %, besonders bevorzugt von mindestens 95 % erhalten.

Die Verbindungen der allgemeinen Formel (II) zeichnen sich durch besondere thermische Stabilität und Hydrolysebeständigkeit aus. Sie können nach dem erfindungsgemäßen Verfahren auf einfachem Wege hergestellt und isoliert werden und sind in hoher Reinheit zugänglich, so dass sie hervorragend als Precursor-Moleküle zur erfindungsgemäßen Herstellung halbleitender Thiophen-Phenylen-Oligomeren der allgemeinen Formel (I) geeignet sind. Überraschend tritt dabei keine Deborierung als Nebenreaktion auf und die erfindungsgemäße Herstellung der Thiophen-Phenylen-Oligomere lässt sich auch unter Bedingungen, wie beispielsweise unter Anwesenheit protischer Lösungsmittel, z.B. Toluol-Wasser-Gemischen, Natriumcarbonat als Base und unter Katalyse mit Tetrakis(triphenylphosphin)palladium durchführen, obwohl bekannt ist, dass Thiophenboronsäuren, wie z.B. 2-Thiophenboronsäure unter diesen Bedingungen schnell zu Borsäure und Thiophen hydrolysieren können und dass dann überwiegend Nebenprodukte erhalten werden (Chemica Scripta, 1984, 23, 120).

Mit dem erfindungsgemäßen Verfahren können somit erstmals ohne aufwändige Reinigungsverfahren Thiophen-Phenylen-Oligomere der allgemeinen Formel (I) mit nur sehr geringen Mengen an Verunreinigungen hergestellt werden. Insbesondere werden die erfindungsgemäß hergestellten Verbindungen der allgemeinen Formel (I) weitgehend frei von schwer abtrennbaren homologen Oligomeren mit höherem oder niedrigerem Molekulargewicht erhalten, so dass eine aufwändige Aufreinigung schwer trennbarer Gemische entfällt.

Bevorzugt werden die Verbindungen der allgemeinen Formel (I) in einer Reinheit von mindestens 95 %, besonders bevorzugt mindestens 99 % erhalten.

Die nach dem erfindungsgemäßen Verfahren hergestellten Verbindungen der allgemeinen Formel (I) sind neutral und halbleitend und eignen sich aufgrund ihrer Reinheit besonders gut für den Einsatz als Halbleiter in aktiven und lichtemittierenden, elektronischen Bauelementen wie Feldeffekt-Transistoren, organischen Lumineszenzdioden, photovoltaischen Zellen, Lasern oder Sensoren. Dazu werden die erfindungsgemäß hergestellten Verbindungen der allgemeinen Formel (I) in Form von Schichten auf geeignete Substrate, zum Beispiel auf mit elektrischen oder elektronischen Strukturen versehene Silizium-Wafer, Polymerfolien oder Glasscheiben aus Lösung oder aus der Gasphase aufgetragen. Für den Auftrag kommen prinzipiell sämtliche dem Fachmann bekannten Auftragsverfahren in Betracht. Beispielsweise können die Verbindungen der allgemeinen Formel (I) aus der Gasphase durch Verdampfen oder Zerstäuben (Sputtern) der neuen Verbindungen und anschließendes Kondensieren oder aus Lösung auf das Substrat aufgetragen werden, wobei man das Lösungsmittel anschließend verdampft. Das Aufbringen aus Lösung kann nach den bekannten Verfahren, beispielsweise durch Sprühen, Tauchen, Drucken und Rakeln, Spin-Coating und durch Ink-Jet-Druck erfolgen. Bevorzugt werden die Verbindungen der allgemeinen Formel (I) aus der Gasphase, z.B. durch Aufdampfen, aufgebracht. Auf diese Weise werden Schichten mit den geringsten Defekten und höchsten Ladungsmobilitäten erhalten.

Halbleitende Schichten, die aus den erfindungsgemäß hergestellten Verbindungen hergestellt werden, zeichnen sich durch hohe Reinheit und folglich geringe Defekte aus.

Weiterhin Gegenstand der vorliegenden Erfindung sind daher Schichten, die nachdem die erfindungsgemäßen Verfahren hergestellten Verbindungen der allgemeinen Formel (I) enthalten und halbleitend sind.

Bevorzugt sind dies Schichten, worin die Verbindungen der allgemeinen Formel (I) solche der allgemeinen Formel (I-a) sind

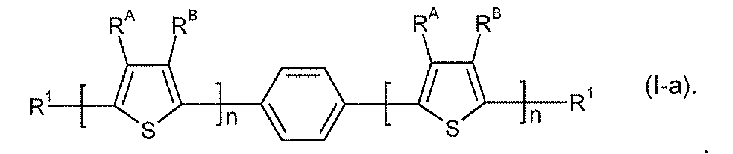

In bevorzugten Ausführungsformen enthalten die erfindungsgemäßen Schichten weniger als 0,5 Gew.-%, bevorzugt weniger als 0,3 Gew,-%, besonderes bevorzugt weniger als 0,05 Gew.-% Halogen.

Die erfindungsgemäßen Schichten können nach dem Aufbringen weiter modifiziert werden, beispielsweise durch eine Temperaturbehandlung, z.B. unter Durchlaufen einer flüssigkristallinen Phase, oder zur Strukturierung z.B. durch Laserablation.

Die erfindungsgemäßen Schichten sind aufgrund ihrer Reinheit hervorragend für die Verwendung in aktiven und lichtemittierenden elektronischen Bauelementen wie Feldeffekt-Transistoren, organischen Lumineszenzdioden, photovoltaischen Zellen, Lasern oder Sensoren geeignet.

### Beispiele

5-(10-Undecenyl)-2,2'-bithiophen, 5-Decyl-2,2':5',2"-terthiophen, 5-Bromo-5'-Ethyl-2,2'-bithiophen und 5-Bromo-5"-ethyl-2,2':5',2"-terthiophen wurden nach bekannten Verfahren hergestellt (Synthesis, 1993, S.1099; Chem. Mater., 1993, Band 5, S. 430; J. Mater. Chem. 2003, Band 13, S. 197).

Alle Reaktionsgefäße wurden vor Gebrauch nach üblicher Schutzgastechnik ausgeheizt und mit Stickstoff geflutet.

### Beispiel 1: Herstellung von 4,4,5,5-Tetramethyl-2-[5'-(10-undecenyl)-2,2'-bithien-5-yl]-1,3,2-dioxaborolan (II-a)

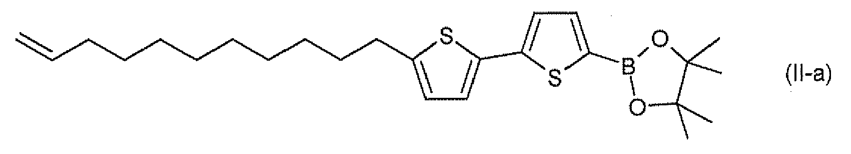

70 ml wasserfreies Tetrahydrofuran (THF) wurden mittels Trockeneis/Aceton auf -74°C heruntergekühlt. Hierzu wurden mit einer Spritze 5.6 ml einer 2.5 M Butyllithiumlösung in Hexan zugetropft. Anschließend wurde ein homogenes Gemisch aus 5-(10-Undecenyl)-2,2'-bithiophen (4.46 g, 14 mmol) in 120 ml wasserfreiem THF zugetropft und 30 min bei -74° nachgerührt. Das Kältebad wurde entfernt, so dass die Temperatur ansteigt. Bei ca. 0°C wurde der Reaktionsansatz wieder heruntergekühlt und bei - 74°C 2-Isopropoxy-4,4,5,5-tetramethyl-1,3,2-dioxaborolan (3.3 ml, 16 mmol) über einer Spritze zugegeben. Es wurde 30 min bei -74°C nachgerührt, das Kühlbad erneut entfernt und die Temperatur auf 20 °C ansteigen gelassen. Das Reaktionsgemisch wurde in 200 ml eisgekühltes Wasser - gemischt mit 15 ml 1 M HCl - gegeben und mit 500 ml Diethylether extrahiert. Die Etherphase wurde abgetrennt, mit Wasser gewaschen, über Na₂SO₄ getrocknet, filtriert und das Lösungsmittel vollständig am Rotationsverdampfer entfernt. Ausbeute: 6.03 g (97 % der Theorie) dunkelblaues, kristallines Produkt (II-a).

### Analysendaten:

GC MS Analyse: M^{•+} 99%, m/e = 444.

¹H NMR (CDCl₃, TMS/ppm): 1.22-1.45 (overlapped peaks with a max at 1.283, 14H), 1.345 (s, 12 H), 1.672 (m, J=7.5 Hz, M = 5, 2H), 2.037 (q, J=7.2 Hz, 2H), 2.781 (t, J=7.3 Hz, 2H), 4.928 (d, J=10.3 Hz, 1 H), 4.991 (d, J=17.1 Hz, 1H), 5.811 (m, 1H), 6.676 (d, J=3.4 Hz, 1H), 7.037 (d, J=3.9 Hz, 1H), 7.152 (d, J=3.9 Hz, 1H), 7.496 (d, J=3.4 Hz, 1H).

### Beispiel 2: Herstellung von 2-(5"-Decyl-2,2':5',2"-terthien-5-yl)-4,4,5,5-tetramethyl-1,3,2-dioxaborolan (II-b)

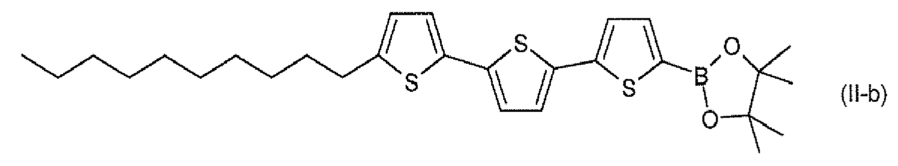

2-(5"-Decyl-2,2':5',2"-terthien-5-yl)-4,4,5,5-tetramethyl-1,3,2-dioxaborolan, 2.4 ml einer 2.5 M Butyllithiumlösung in Hexan, 5-Decyl-2,2':5',2"-terthiophen (2.33 g, 6 mmol) und 2-Isopropoxy-4,4,5,5-tetrametliyl-1,3,2-dioxaborolan (1.4 ml, 7 mmol) werden wie in Beispiel 1 beschrieben in 80 ml wasserfreiem THF umgesetzt und das entstehende Produkt isoliert. Ausbeute: 3.02 g (98 % der Theorie) olivgrünes, festes Produkt (II-b).

### Analysendaten:

EI MS Analyse: M^{•+} ca. 90%, m/e = 514.

¹H NMR (CDCl₃, TMS/ppm): 0.880 (3H, t, J=6.9 Hz), 1.20-1.45 (overlapped peaks, 14 H), 1.351 (s, 12 H), 1.678 (2H, m, J=7.5 Hz), 2.787 (2H, t, J=7.6 Hz), 6.679 (1H, d, J=3.4 Hz), 6.988 (2H, dd, J1=3.9 Hz, J2=3.9 Hz), 7.111 (1H, d, J=3.9 Hz), 7.209 (1H, d, J=3.9 Hz), 7.516 (1H, d, J=3.4 Hz).

### Beispiel 3: Herstellung von 5-Decyl-5"-[4-(5"-decyl-2,2':5',2"-terthien-5-yl)phenyl]-2,2':5',2"-terthiophen (I-a-1)

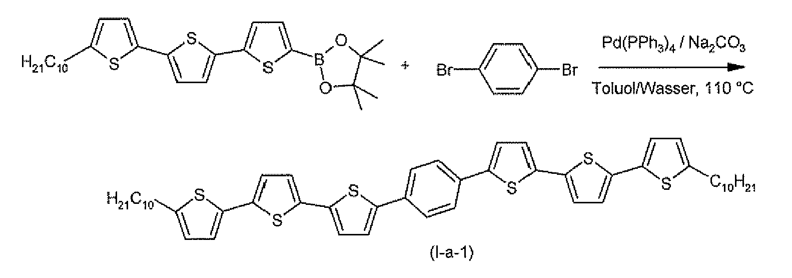

1,4-Dibrombenzol (142 mg, 0.6 mmol) wurde vorgelegt und unter Schutzgas Tetrakis(triphenylphosphin)palladium Pd(PPh₃)₄ (46 mg, 0.04 mmol) dazugegeben. Es wurde eine Lösung von 2-(5"-Decyl-2,2':5',2"-terthien-5-yl)-4,4,5,5-tetramethyl-1,3,2-dioxaborolan (II-b) (865 mg, 1.7 mmol) in 20 ml wasserfreiem Toluol und 5 ml einer 2 M wässrigen Na₂CO₃-Lösung vorbereitet und mit Stickstoff desoxidiert. Beide Lösungen wurden mit Spritzen zum Reaktionsansatz gegeben und das Reaktionsgemisch anschließend für 20 Stunden unter Rückfluss erhitzt. Das Reaktionsgemisch wurde nach Abkühlen in ein Gemisch aus 200 ml Wasser, 80 ml 1 M HCl und 300 ml Toluol gegeben. Nach dreimaligem Ausschütteln mit je 100 ml Wasser wurde die organische Phase über einen G4 Glasfilter filtriert und der Filterrückstand getrocknet. Ausbeute: 404 mg (79 % der Theorie) orangefarbenes Pulver.

### Analysendaten:

EI MS Analyse: M^{•+} 99+%, m/e = 850.3

EI MS Analyse (nach Aufreinigung durch Sublimation bei 0,25 mBar, 320°C)): M^{•+} 100%, m/e = 850.3.

Schmelzverhalten (°C): K 310 I (K = kristallin, I = isotrop flüssig).

Das Schmelzverhalten wurden bestimmt mit einem DSC (Differential Scanning Calorimeter) Mettler TA-4000 Thermosystem, Scanning rate 10K/min.

## Patentansprüche

1. Halbleitende Schichten enthaltend Verbindungen der allgemeinen Formel (I) 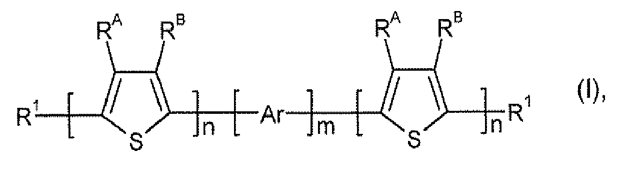 worin
m für eine ganze Zahl von 1 bis 5 steht,
Ar für gegebenenfalls substituiertes 1,4-Phenylen und/oder 2,7-Fluorenylen steht,
R¹ für H oder eine gegebenenfalls subsituierte lineare oder verzweigte C₁-C₂₀-Alkylgruppe, eine gegebenenfalls durch ein(e) oder mehrere O- oder S-Atome, Silylen-, Phosphonoyl- oder Phosphorylgruppen unterbrochene lineare C₁-C₂₀-Alkylgruppe oder eine C₃-C₂₀-Alkenylgruppe steht,
R^{A}, R^{B} unabhängig voneinander jeweils für H oder eine gegebenenfalls substituierte und/oder gegebenenfalls mit bis zu 10 Sauerstoffatomen unterbrochene lineare oder verzweigte C₁-C₂₀-Alkylgruppe, eine gegebenenfalls substituierte C₁-C₂₀-Alkoxygruppe oder gemeinsam für eine gegebenenfalls substituierte C₁-C₆-Dioxyalkylengruppe stehen und
n für 2, 3 oder 4 steht,
**dadurch gekennzeichnet, dass** die Verbindungen der allgemeinen Formel (I) nach folgendem Verfahren, insbesondere nach dem Prinzip der Suzuki-Kupplung, hergestellt werden:
Umsetzung einer Verbindung der allgemeinen Formel (II) 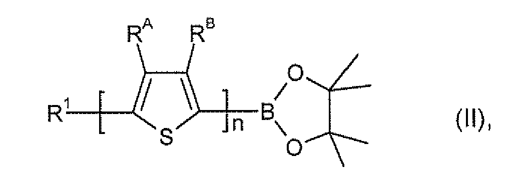
worin
n, R¹, R^{A} und R^{B} die oben für die allgemeine Formel (I) genannte Bedeutung haben,
mit einer Verbindung der allgemeinen Formel (III) 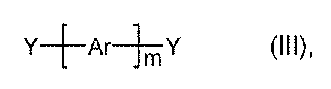 worin
m und Ar die für die allgemeine Formel (I) genannte Bedeutung haben und
Y für einen Rest Cl, Br, I oder O-SO₂-R^{C} steht, wobei R^{C} für einen Phenyl-, Tolyl-, Methyl- oder Trifluormethylrest steht,
gegebenenfalls in Anwesenheit von Katalysatoren, gegebenenfalls in Lösung und gegebenenfalls bei erhöhter Temperatur.

2. Halbleitende Schichten gemäß Anspruch 1 enthaltend Verbindungen der allgemeinen Formel (I-a) 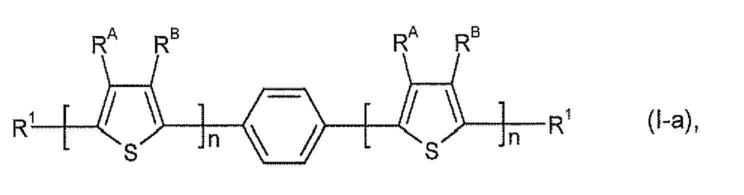 worin
n, R¹, R^{A} und R^{B} die in Anspruch 1 genannte Bedeutung haben,
**dadurch gekennzeichnet, dass** bei dem Verfahren zur Herstellung der Verbindungen der allgemeinen Formel (I-a) eine Verbindung der allgemeinen Formel (II) mit einer Verbindung der allgemeinen Formel (III-a) 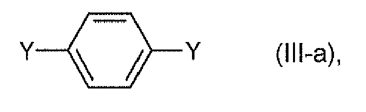 worin
Y die in Anspruch 1 genannte Bedeutung hat,
umgesetzt wird.

3. Halbleitende Schichten gemäß Anspruch 1 oder 2, **dadurch gekennzeichnet, dass** R^{A} und R^{B} für H stehen.

4. Halbleitende Schichten gemäß wenigstens einem der Ansprüche 1 bis 3, **dadurch gekennzeichnet, dass** bei den Verfahren zur Herstellung der Verbindungen gemäß der allgemeinen Formel (I) oder (I-a) die Umsetzung in Gegenwart wenigstens eines Katalysators enthaltend ein Metall der VIII. Nebengruppe des Periodensystems der Elemente erfolgt.

5. Halbleitende Schichten gemäß wenigstens einem der Ansprüche 1 bis 4, **dadurch gekennzeichnet, dass** bei den Verfahren zur Herstellung der Verbindungen gemäß der allgemeinen Formel (I) oder (I-a) die Umsetzung in Gegenwart wenigstens einer Base erfolgt.

6. Halbleitende Schichten gemäß wenigstens einem der Ansprüche 1 bis 5, **dadurch gekennzeichnet, dass** bei den Verfahren zur Herstellung der Verbindungen gemäß der allgemeinen Formel (I) oder (I-a) die Umsetzung in Gegenwart wenigstens eines organischen Lösungsmittels erfolgt.

7. Verwendung der Schichten gemäß wenigstens einem der Ansprüche 1 bis 6 als halbleitende Schichten in aktiven und lichtemittierenden elektronischen Bauelementen wie Feldeffekt-Transistoren, organischen Lumineszenzdioden, photovoltaischen Zellen, Lasern oder Sensoren.

8. Verfahren zur Herstellung der Schichten gemäß wenigstens einem Ansprüche 1 bis 6, **dadurch gekennzeichnet, dass** die Verbindungen gemäß der allgemeinen Formel (I) und der allgemeinen Formel (I-a) aus einer Lösung der Verbindungen in einem Lösungsmittel durch Aufbringen der Lösung und Verdampfen des Lösungsmittels oder aus der Gasphase durch Verdampfen oder Zerstäuben der Verbindungen und anschließendes Kondensieren auf ein geeignetes Substrat aufgebracht werden.
